# EUROPEAN PATENT APPLICATION

(11) **EP 2 112 142 A1**
(43) Date of publication of application: **28.10.2009**
(21) Application number: 08425287.3
(22) Date of filing: 24.04.2008
(51) Int. Cl.: C07D 261/20, A61K 31/423, A61P 25/00

(54) **Process for preparing a crystalline form compound of 3-benzyl-2-methyl-2,3,3a,4,5,6,7,7a-octahydrobenzo[d]isoxazol-4-one**

(71) Applicant: Abiogen Pharma S.p.A., 56014 Località Ospedaletto (PI) (IT)
(72) Inventor: Basagni, Simone, 56124 Pisa (IT); Neggiani, Fabio, 56123 Pisa (IT); Politi, Barbara, 57124 Livorno (IT); Napolitano, Elio, 56127 Pisa (IT)
(74) Representative: Cattaneo, Elisabetta

(57) **Abstract**

A process for preparing a crystalline form compound of 3-benzyl-2-methyl-2,3,3a,4,5,6,7,7a-octahydrobenzo[d]isoxazol-4-one is described, comprising the step of reacting 3-benzyl-2-methyl-2,3,3a,4,5,6,7,7a-octahydrobenzo[d]isoxazol-4-one and fumaric acid in one or more solvents, wherein at least one solvent is a solvent having a carbon atom number of from 3 to 6, said solvent being non-halogenated and having a dielectric constant ε in the range from 4 to 25. The process of the invention enables to obtain a co-crystal comprising 3-benzyl-2-methyl-2,3,3a,4,5,6,7,7a-octahydrobenzo[d]isoxazol-4-one and fumaric acid.

## Description

### FIELD OF THE INVENTION

The present invention concerns a process for preparing a crystalline form compound of 3-benzyl-2-methyl-2,3,3a,4,5,6,7,7a-octahydrobenzo[d]isoxazol-4-one of formula: 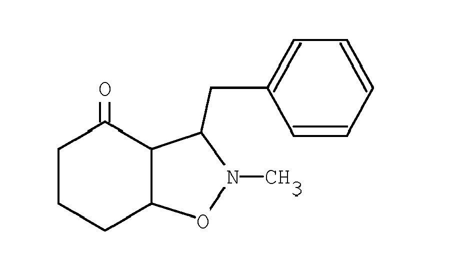 in all its stereochemical configurations, a co-crystal obtained by said process comprising 3-benzyl-2-methyl-2,3,3a,4,5,6,7,7a-octahydrobenzo[d]isoxazol-4-one and fumaric acid, and its use for the treatment of mood disorders, disorders of anxiety, depression, convulsive conditions, in the improvement of learning ability, in the reversal of amnesia, in resolving abstinence syndrome from medicaments and drugs.

### STATE OF THE ART

The compound rel-(3R,3aS,7aS)-3-benzyl-2-methyl-2,3,3a,4,5,6,7,7a-octahydrobenzo[d]isoxazol-4-one, also known as BTG 1640, is described in international patent application WO93/17004 and belongs to a new family of psychoactive agents.

According to this document, the compound BTG 1640 is prepared as a yellow oil then salified as a hydrochloride salt.

Said preparation, which comprises the use of an oily free base for forming the hydrochloride salt, requires a complicated crystallization and purification stage for obtaining a pharmaceutical grade salt.

Thus, there is still a need for a BTG 1640 compound in crystalline form which is easy to prepare, i.e. that does not require difficult and lengthy crystallizations and hence can be easily scaled-up to industrial levels.

The object of the present invention is therefore to provide the compound BTG 1640 in crystalline form which responds to the need for an industrially scalable process.

### SUMMARY

Since the hydrochloride salt of BTG 1640 not only had preparation problems, but it was also unstable at temperatures above 30ºC, the inventors of the present invention have turned their attention to alternative preparations to BTG 1640 hydrochloride.

The aforementioned object was hence attained through the selection of fumaric acid and the selection of reaction solvent(s).

The invention therefore concerns a process for preparing a crystalline form compound of 3-benzyl-2-methyl-2,3,3a,4,5,6,7,7a-octahydrobenzo[d]isoxazol-4-one which comprises the step of reacting 3-benzyl-2-methyl-2,3,3a,4,5,6,7,7a-octahydrobenzo[d]isoxazol-4-one with fumaric acid in one or more solvents, wherein at least one solvent is a solvent having a carbon atom number of from 3 to 6, said solvent being non-halogenated and having a dielectric constant ε in the range from 4 to 25.

In a preferred aspect, the invention concerns a process for preparing a crystalline form compound of 3-benzyl-2-methyl-2,3,3a,4,5,6,7,7a-octahydrobenzo[d]isoxazol-4-one which comprises the step of reacting 3-benzyl-2-methyl-2,3,3a,4,5,6,7,7a-octahydrobenzo[d]isoxazol-4-one with fumaric acid in a 2:1 molar ratio, in one or more solvents, wherein at least one solvent is a solvent having a carbon atom number of from 3 to 6, said solvent being non-halogenated and having a dielectric constant ε in the range from 4 to 25.

The inventors of the present invention have surprisingly found that the crystalline form compound of BTG 1640 obtained by the process of the invention is a co-crystal.

Another aspect of the invention, therefore, concerns a co-crystal comprising 3-benzyl-2-methyl-2,3,3a,4,5,6,7,7a-octahydrobenzo[d]isoxazol-4-one and fumaric acid.

In a further aspect of the invention, the co-crystal comprising 3-benzyl-2-methyl-2,3,3a,4,5,6,7,7a-octahydrobenzo[d]isoxazol-4-one and fumaric acid is used as a medicament.

In yet a further aspect of the invention, the co-crystal comprising 3-benzyl-2-methyl-2,3,3a,4,5,6,7,7a-octahydrobenzo[d]isoxazol-4-one and fumaric acid is used for the manufacture of a medicament for treating mood disorders, disorders of anxiety, depression, convulsive conditions, in the improvement of learning ability, in the reversal of amnesia, in resolving abstinence syndrome from medicaments and drugs.

### DESCRIPTION OF THE FIGURES

The characteristics and advantages of the invention will be evident from the detailed description that follows and from the accompanying figures, in which:
- Figure 1 shows the observed experimental X-ray powder diffractogram of the 3-benzyl-2-methyl-2,3,3a,4,5,6,7,7a-octahydrobenzo[d]isoxazol-4-one and fumaric acid co-crystal of the invention;
- Figure 2 shows the experimental diffractogram calculated from the information obtained from X-ray diffractography on a single crystal of the 3-benzyl-2-methyl-2,3,3a,4,5,6,7,7a-octahydrobenzo[d]isoxazol-4-one and fumaric acid co-crystal of the invention; and
- Figure 3 shows the table containing a list of the characteristic peaks of the calculated experimental diffractogram shown in figure 2.

### DETAILED DESCRIPTION OF THE INVENTION

The invention therefore concerns a process for preparing a crystalline form compound of 3-benzyl-2-methyl-2,3,3a,4,5,6,7,7a-octahydrobenzo[d]isoxazol-4-one which comprises the step of reacting 3-benzyl-2-methyl-2,3,3a,4,5,6,7,7a-octahydrobenzo[d]isoxazol-4-one with fumaric acid in one or more solvents, wherein at least one solvent is a solvent having a carbon atom number of from 3 to 6, said solvent being non-halogenated and having a dielectric constant ε in the range from 4 to 25.

The reaction between 3-benzyl-2-methyl-2,3,3a,4,5,6,7,7a-octahydrobenzo[d]isoxazol-4-one and fumaric acid takes place in the presence of one or more solvents. In this respect, the inventors of the present invention have shown that they have obtained a compound in crystalline form by means of a process suitable for industrial scale-up, through the selection of at least one reaction solvent according to specific physical and structural characteristics. Said reaction solvent is hence a non-halogenated solvent, having a carbon atom number of from 3 to 6 and having a dielectric constant ε in the range from 4 to 25. The at least one solvent of the invention is preferably selected from the group consisting of diethylether, t-butyl-methyl-ether (MTBE), 1,2-dimethoxyethane, tetrahydrofuran (THF), diisopropylether, 4-methyl-2-pentanone, 2-methoxyethanol, 2-butanol, 2-methyl-1-propanol, 2-propanol, 2-butanone, 1-propanol, 1-butanol, acetone.

In the most preferred embodiment of the invention the at least one reaction solvent is selected from the group consisting of t-butyl-methyl-ether (MTBE), tetrahydrofuran (THF), 1-butanol, acetone. In said embodiment, advantageously, the yield of the crystalline form compound is in the range from 70% to 99%. Advantageously, by using at least one solvent of the invention selected from the group consisting of t-butyl-methyl-ether (MTBE), tetrahydrofuran (THF), 1-butanol, acetone, the reaction between BTG 1640 and fumaric acid can be conducted with decidedly moderate amounts of solvents, i.e. a ratio of solvent volume/mmol of reacting fumaric acid in the range from 2 to 13, and preferably in the range from 2.8 to 12.2.

In a preferred aspect, the invention concerns a process for preparing a crystalline form compound of 3-benzyl-2-methyl-2,3,3a,4,5,6,7,7a-octahydrobenzo[d]isoxazol-4-one which comprises the step of reacting 3-benzyl-2-methyl-2,3,3a,4,5,6,7,7a-octahydrobenzo[d]isoxazol-4-one with fumaric acid in a 2:1 molar ratio, in one or more solvents, wherein at least one solvent is a solvent having a carbon atom number of from 3 to 6, said solvent being non-halogenated and having a dielectric constant ε in the range from 4 to 25.

In the process of the invention fumaric acid is hence reacted with the BTG 1640 free base, preferably in a 1:2 ratio.

Preferably in the process of the invention fumaric acid is added under reflux to the solution consisting of the BTG 1640 free base dissolved in one or more solvents, said reflux conditions being maintained until a clear solution is obtained. The solution is then cooled to a temperature in the range from room temperature to - 25º C for a time varying from 3 to 24 hours. Advantageously, if the at least one reaction solvent is methyl-t-butyl-ether (MTBE) or 1-butanol, yields higher than 70% can be obtained even at room temperature. The compound in crystalline form which separates as a colourless compound can be optionally subjected to further purification cycles according to the known art techniques. The mother waters can possibly be evaporated and subjected to prolonged cooling with the aim of recovering more of the BTG 1640 and fumaric acid compound in crystalline form.

The compound in crystalline form which separates from the process of the invention is a co-crystal comprising fumaric acid and BTG 1640 free base.

As it will be demonstrated in the experimental part, the co-crystal of the invention is a crystalline molecular complex, i.e. a combination of the two molecules BTG 1640 and fumaric acid spatially disposed to create a single crystalline form.

The co-crystal of the invention was characterized by X-ray diffractometry carried out on both the powders and the single crystal.

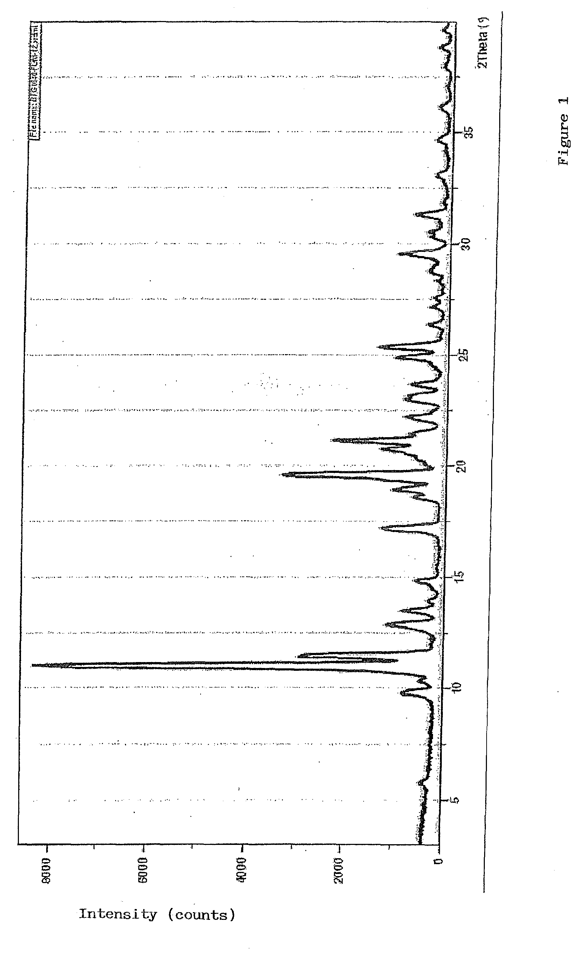

X-ray powder diffractometry was used to obtain the observed experimental diffractogram shown in Figure 1, from which it is inferred that the co-crystal of the invention exhibits peaks at the diffraction degrees (±0.2°2θ) shown in the following Table 1:

**Table 1**

| | | | | |
|---|---|---|---|---|
| 5.79 | 14.82 | 22.23 | 27.56 | 34.56 |
| 9.89 | 17.27 | 22.96 | 28.73 | 34.68 |
| 10.31 | 18.57 | 23.18 | 29.56 | 36.18 |
| 10.93 | 18.91 | 23.65 | 30.21 | 37.95 |
| 11.39 | 19.50 | 24.82 | 30.53 | 38.80 |
| 12.88 | 20.73 | 25.27 | 31.33 | 39.62 |
| 13.49 | 21.16 | 26.33 | 31.87 | |
| 13.98 | 21.45 | 27.14 | 33.00 | |

More specifically, the diffractogram relating to the co-crystal of the invention exhibits characteristic peaks at the following diffractometer angles:
5.79 °2θ
9.89 °2θ
10.93 °2θ
17.27 °2θ
19.50 °2θ
22.23 °2θ

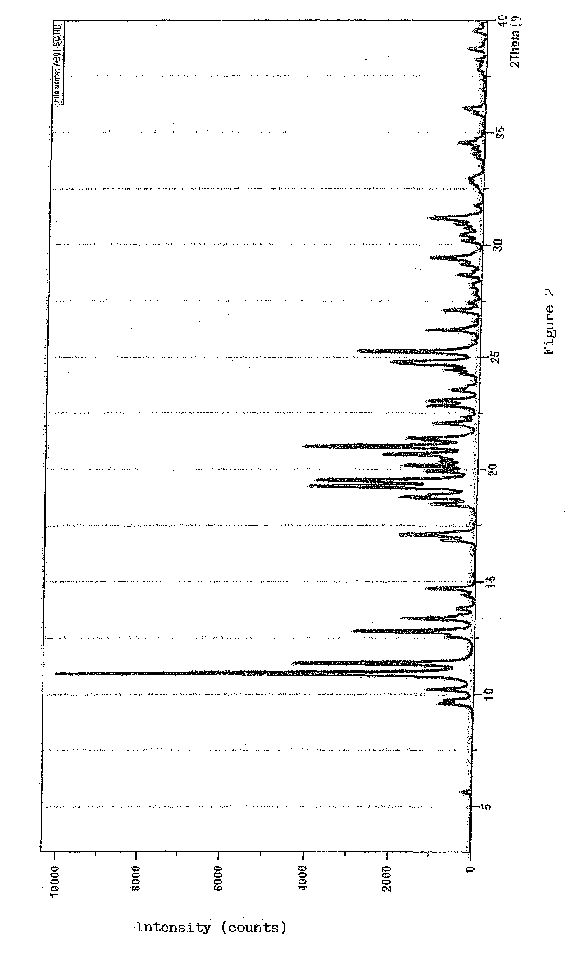

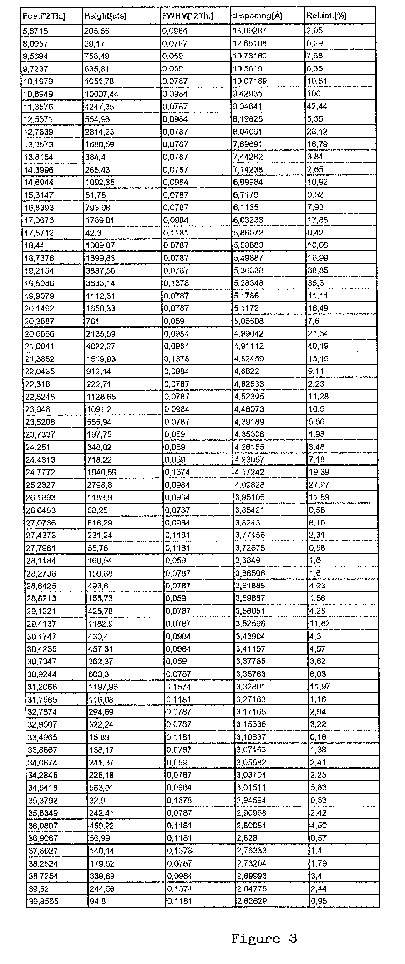

From the X-ray diffractometry on the single crystal, information on the structure and on the interatomic distances of the molecules involved was obtained, which confirmed the fact that the crystalline solid is a co-crystal consisting of two molecules of BTG 1640 and one of fumaric acid. Calculations on the data obtained from the diffraction analysis on the single crystal of BTG 1640 and fumaric acid co-crystal, have generated the calculated experimental diffractogram being devoid of any of the imperfections and background noise typical of microcrystalline powders. The calculated experimental diffractogram is given in Figure 2, the characteristic peaks of which are given in the subsequent Figure 3. According to the invention, the co-crystal is therefore obtained by a simple procedure, easily scalable to industrial levels and avoiding the use of lengthy and costly crystallization and purification steps, to obtain high yields of a stable pharmaceutical grade crystalline form.

The co-crystal comprising BTG 1640 and fumaric acid of the invention can be used as a medicament.

It can then be combined with a pharmaceutically acceptable carrier and, optionally, with suitable excipients, to obtain pharmaceutical compositions. The term "pharmaceutically acceptable carrier" includes solvents, diluents and the like which are used in the administration of the co-crystals of the invention.

Said pharmaceutical compositions can be parenterally, orally or topically administered.

Compositions of the present invention suitable for oral administration will be conveniently in the form of discrete units such as tablets, capsules, cachets, as powders or granules, or as a suspension in a liquid.

More preferably, the compositions of the invention for oral administration will be in the form of tablets.

The tablets will preferably comprise an amount from 1 to 100 mg, even more preferably from 1 to 50 mg, of the co-crystal comprising fumaric acid and BTG 1640.

Preferably the tablets will contain from 1.9% to 41.1 % by weight of the co-crystal comprising BTG 1640 and fumaric acid and even more preferably the co-crystal comprising BTG 1640 and fumaric acid will constitute from 2.2% to 36% of the total tablet weight.

The tablets could also contain suitable excipients of common pharmaceutical use such as pre-gelatinized starch, microcrystalline cellulose, sodium starch glycolate, talc, lactose, magnesium stearate, sucrose, stearic acid, mannitol.

Compositions for parenteral administration will conveniently comprise sterile preparations.

Preparations for parenteral administration will preferably comprise an amount from 0.1 to 100 mg of co-crystal comprising fumaric acid and BTG 1640.

Compositions for topical administration will be conveniently in the form of creams, pastes, poultices, oils, ointments, emulsions, foams, gels, drops, aqueous solutions, spray solutions and transdermal patches.

Preparations for topical administration will preferably comprise an amount from 1 to 100 mg of co-crystal comprising fumaric acid and BTG 1640.

The co-crystal of the invention can be used for the production of a medicament for the treatment of mood disorders, disorders of anxiety, depression, convulsive conditions, in the improvement of learning ability, in the reversal of amnesia, in resolving abstinence syndrome from medicaments and drugs.

The invention will now be described in greater detail in the following examples, given by way of non-limiting illustration of the invention, relative to the process of the invention and to the characterization of the co-crystal obtained by the process.

### EXAMPLES

### Example 1

### Process for preparing BTG 1640 and fumaric acid co-crystal in acetone

0.8035 g of BTG 1640 free base (3.28 mmol) were placed in a 50 ml flask containing 10 ml of acetone; 0.190 g (1.64 mmol) of anhydrous fumaric acid were then added under reflux to the solution thus obtained. Reflux conditions were maintained until a clear solution was obtained. After cooling for 15 hours at a temperature of -20ºC, the BTG 1640 and fumaric acid co-crystal separated from the solution (melting point between 110 and 120ºC) with a yield of 80%.

### Example 2

### Process for preparing the BTG 1640 and fumaric acid co-crystal in tert-butyl-methyl-ether (MTBE)

0.8036 g of BTG 1640 free base (3.28 mmol) were placed in a 50 ml flask containing 20 ml of tert-butylmethylether (MTBE); 0.190 g (1.64 mmol) of anhydrous fumaric acid were then added under reflux to the solution thus obtained. Reflux conditions were maintained until a clear solution was obtained. After cooling for 15 hours at a temperature of -20ºC, the BTG 1640 and fumaric acid co-crystal separated from the solution (melting point between 110 and 120ºC) with a yield of 92%.

### Example 3

### Process for preparing the BTG 1640 and fumaric acid co-crystal in tert-butyl-methyl-ether (MTBE)

0.8036 g of BTG 1640 free base (3.28 mmol) were placed in a 50 ml flask containing 20 ml of tert-butylmethylether (MTBE); 0.190 g (1.64 mmol) of anhydrous fumaric acid were then added under reflux to the solution thus obtained. Reflux conditions were maintained until a clear solution was obtained. After cooling for 15 hours at a temperature of 4ºC, the BTG 1640 and fumaric acid co-crystal separated from the solution (melting point between 110 and 120ºC) with a yield of 80%.

### Example 4

### Process for preparing the BTG 1640 and fumaric acid co-crystal in tert-butyl-methyl-ether (MTBE)

0.8035 g of BTG 1640 free base (3.28 mmol) were placed in a 50 ml flask containing 20 ml of tert-butylmethylether (MTBE); 0.190 g (1.64 mmol) of anhydrous fumaric acid were then added under reflux to the solution thus obtained. Reflux conditions were maintained until a clear solution was obtained. After cooling for 15 hours at room temperature, the BTG 1640 and fumaric acid co-crystal separated from the solution (melting point between 110 and 120ºC) with a yield of 73%.

### Example 5

### Process for preparing the BTG 1640 and fumaric acid co-crystal in diisopropylether

0.624 g of BTG 1640 free base (2.54 mmol) were placed in a 100 ml flask containing 60 ml of diisopropylether; 0.147 g (1.27 mmol) of anhydrous fumaric acid were then added under reflux to the solution thus obtained. Reflux conditions were maintained until a clear solution was obtained. After cooling for 5 hours at a temperature of -20ºC, the BTG 1640 and fumaric acid co-crystal separated from the solution (melting point between 110 and 120ºC) with a yield of 74%.

### Example 6

### Process for preparing the BTG 1640 and fumaric acid co-crystal in diisopropylether

0.624 g of BTG 1640 free base (2.54 mmol) were placed in a 100 ml flask containing 60 ml of diisopropylether; 0.294 g (2.54 mmol) of anhydrous fumaric acid were then added under reflux to the solution thus obtained. Reflux conditions were maintained for 2 hours after which the undissolved fumaric acid was separated by filtration. The clear solution was cooled for 5 hours at a temperature of -20ºC, and the BTG 1640 and fumaric acid co-crystal (melting point between 110 and 120ºC) separated with a yield of 74%.

### Example 7

### Process for preparing the BTG 1640 and fumaric acid co-crystal in diisopropylether

0.703 g of BTG 1640 free base (2.87 mmol) were placed in a 100 ml flask containing 60 ml of diisopropylether; 0.294 g (2.54 mmol) of anhydrous fumaric acid were then added under reflux to the solution thus obtained. Reflux conditions were maintained for 2 hours after which the undissolved fumaric acid was separated by filtration. The clear solution was cooled for 5 hours at a temperature of -20ºC, and the BTG 1640 and fumaric acid co-crystal (melting point between 110 and 120ºC) separated with a yield of 75%.

From examples 5-7 the inventors of the present invention have shown there is no advantage in terms of yield by operating under conditions where one or other of the reagents is in excess, i.e. under conditions in which the molar ratio of BTG 1640 to fumaric acid is other than 2:1. Advantageously in the process of the present invention the reagents BTG 1640 and fumaric acid will hence be reacted respectively in the stoichiometric ratio of about 2:1.

### Example 8

### Process for preparing the BTG 1640 and fumaric acid co-crystal in tetrahydrofuran

0.854 g of BTG 1640 free base (3.48 mmol) were placed in a 50 ml flask containing 10 ml of tetrahydrofuran; 0.202 g (1.74 mmol) of anhydrous fumaric acid were then added under reflux to the solution thus obtained. Reflux conditions were maintained until a clear solution was obtained. After cooling for 15 hours at a temperature of -20ºC, the BTG 1640 and fumaric acid co-crystal separated from the solution (melting point between 110 and 120ºC) with a yield of 71 %.

### Example 9

### Process for preparing the BTG 1640 and fumaric acid co-crystal in 1-butanol

0.820 g of BTG 1640 free base (3.34 mmol) were placed in a 50 ml flask containing 10 ml of 1-butanol; 0.194 g (1.67 mmol) of anhydrous fumaric acid were then added under reflux to the solution thus obtained. Reflux conditions were maintained until a clear solution was obtained. After cooling for 4 hours at a temperature of -20ºC, the BTG 1640 and fumaric acid co-crystal separated from the solution (melting point between 110 and 120ºC) with a yield of 99%.

### Example 10

### Characterization of the co-crystal through X-ray diffractometry on the single crystal

The co-crystal obtained in example 1 was analysed to determine its structure.

Specifically the colourless needle crystal of example 1, being 0.2 x 0.2 x 0.3 mm in size, was mounted on a glass fibre in a random orientation. The crystallographic data were collected at room temperature using a Nonius CAD-4 diffractometer, Mo-kα radiation, α=0.71073 Å, with a graphite monochromator.

The cell parameters and an orientation matrix for data collection were obtained by the least-squares method using the setting angles of 25 reflections in the range 7°<θ<15°.

The space group was determined by means of the XPREP programme. The spacial group was P21/c. The structure was solved by direct methods and refined using the full-matrix least-squares method on *F*² with the SHELX-97 programme. The crystallographic data obtained are summarized in Table 2 below.

**Table 2: Crystallographic data of the compound of the invention in crystalline form.**

| | | |
|---|---|---|
| Empirical Formula | C₃₄H₄₂N₂O₈ | |
| Molecular weight | 606.70 | |
| Temperature | 20°C | |
| Wavelength | 0.71073 Å | |
| Space group | Monocline, P21/c | |
| Unit cell dimensions | a=10.9361(6) Å | alpha= 90° |
| | b=9.5073(6) Å | beta=93.8430(10) ° |
| | c=31.209(2) Å | gamma=90° |
| Volume | 3237.6(3) A³ | |
| Z, calculated density | 4, 1.245 Mg/m³ | |
| Absorption coefficient | 0.089 mm⁻¹ | |
| F(000) | 1296 | |
| Crystal size | 0.2 x 0.2 x 0.3 mm | |
| Theta range for data collection | 1.31 - 23.12 ° | |
| Limiting indices | -12≤h≤12, -10≤k≤10, -34≤I≤34 | |
| Reflections collected/unique | 19937/9067 [R(int)=0.0169] | |
| Completeness to theta | 23.12 99.1 % | |
| Refinement method | full-matrix least-squares on *F²* | |
| Data/restraints/parameters | 9067/0/762 | |
| Goodness of fit on *F*² | 1.018 | |
| Final R indices [I>2sigma(I)] | R1=0.0431, wR2=0.1053 | |
| R indices (all data) | R1=0.0575, wR2=0.1163 | |
| Extinction coefficient | 0.0028 (4) | |
| Largest diffractometric peak and hole | 0.298 e -0.282 eA⁻³ | |

The results of the crystallographic analysis have demonstrated that in samples of the substance obtained in example 1, there is no transfer of hydrogen between the fumaric acid carboxyl groups and the nitrogen atom of the BTG 1640 free base confirming that the compound of the invention is a co-crystal of fumaric acid and BTG 1640. Figures 2 and 3 show respectively the calculated experimental diffractogram for the co-crystal and the corresponding table listing the values of the various peaks in said diffractogram.

### Example 11

### Characterization of the co-crystal by determining the X-ray powder diffractogram

An X-ray powder diffractogram was obtained from the same sample as example 10 by the X'Pert Pro analytical automated diffractometer equipped with X'Celerator, CuKα, using glass sample holders and 150 mg of substance, setting the voltage and amperage to 40kV and 40mA respectively. The programme used for collecting data was set to obtain the data within the 2 theta range from 3º to 40º.

The observed experimental diffractogram is shown in Figure 1.

The peaks are given in table 1 below:

**Table 1**

| | | | | |
|---|---|---|---|---|
| 5.79 | 14.82 | 22.23 | 27.56 | 34.56 |
| 9.89 | 17.27 | 22.96 | 28.73 | 34.68 |
| 10.31 | 18.57 | 23.18 | 29.56 | 36.18 |
| 10.93 | 18.91 | 23.65 | 30.21 | 37.95 |
| 11.39 | 19.50 | 24.82 | 30.53 | 38.80 |
| 12.88 | 20.73 | 25.27 | 31.33 | 39.62 |
| 13.49 | 21.16 | 26.33 | 31.87 | |
| 13.98 | 21.45 | 27.14 | 33.00 | |

### Example 12

The same analyses as in examples 10 and 11 were carried out on the samples obtained from preparative examples 2-9. The obtained results are in agreement with the results given in examples 10 and 11, confirming the fact that all the preparative conditions of examples 1-9 have resulted in the BTG 1640 and fumaric acid co-crystal according to the invention.

The process of the invention, being simple and of immediate industrial scale-up, has hence provided a new crystalline form which is a co-crystal comprising BTG 1640 and fumaric acid.

## Claims

1. Process for preparing a crystalline form compound of 3-benzyl-2-methyl-2,3,3a,4,5,6,7,7a-octahydrobenzo[d]isoxazol-4-one comprising the step of reacting 3-benzyl-2-methyl-2,3,3a,4,5,6,7,7a-octahydrobenzo[d]isoxazol-4-one with fumaric acid in one or more solvents, wherein at least one solvent is a solvent having a carbon atom number of from 3 to 6, said solvent being non-halogenated and having a dielectric constant ε in the range from 4 to 25.

2. Process according to claim 1 wherein 3-benzyl-2-methyl-2,3,3a,4,5,6,7,7a-octahydrobenzo[d]isoxazol-4-one is reacted with fumaric acid in a molar ratio of about 2:1.

3. Process according to claims 1 or 2 wherein the at least one solvent is selected from the group consisting of diethylether, t-butyl-methyl-ether (MTBE), 1,2-dimethoxyethane, tetrahydrofuran (THF), diisopropylether, 4-methyl-2-pentanone, 2-methoxyethanol, 2-butanol, 2-methyl-1-propanol, 2-propanol, 2-butanone, 1-propanol, 1-butanol, acetone.

4. Process according to claim 3 wherein the at least one reaction solvent is selected from the group consisting of t-butyl-methyl-ether (MTBE), tetrahydrofuran (THF), 1-butanol, acetone.

5. Process according to claim 4 wherein the yield of the crystalline form compound is in the range 70% to 99%.

6. Process according to claim 4 wherein the reaction between BTG 1640 and fumaric acid is conducted with a ratio of solvent volume/mmol of fumaric acid in the range from 2 to 13, and preferably in the range from 2.8 to 12.2.

7. Process according to any one of claims from 1 to 6 wherein the fumaric acid is added under solvent reflux conditions until a clear solution is obtained.

8. Process according to any one of claims from 1 to 7 wherein the solution containing the fumaric acid and BTG 1640 is cooled at a temperature in the range from room temperature to -25ºC for a time varying from 3 to 24 hours.

9. Process according to claim 4 wherein when the at least one reaction solvent is methyl-t-butyl-ether (MTBE) or 1-butanol, the yields are higher than 70% even at room temperature.

10. Co-crystal comprising fumaric acid and 3-benzyl-2-methyl-2,3,3a,4,5,6,7,7a-octahydrobenzo[d]isoxazol-4-one.

11. Co-crystal according to claim 10 wherein the co-crystal exhibits an X-ray powder diffractogram having characteristic peaks expressed in diffraction degrees (±0.2°2θ) at 5.79, 9.89, 10.93, 17.27, 19.50 and 22.23 ±0.2°2θ.

12. Co-crystal according to claim 10 wherein the co-crystal exhibits an X-ray powder diffractogram having the following peaks expressed in diffraction degrees (±0.2 °2θ):
| | | | | |
|---|---|---|---|---|
| 5.79 | 14.82 | 22.23 | 27.56 | 34.56 |
| 9.89 | 17.27 | 22.96 | 28.73 | 34.68 |
| 10.31 | 18.57 | 23.18 | 29.56 | 36.18 |
| 10.93 | 18.91 | 23.65 | 30.21 | 37.95 |
| 11.39 | 19.50 | 24.82 | 30.53 | 38.80 |
| 12.88 | 20.73 | 25.27 | 31.33 | 39.62 |
| 13.49 | 21.16 | 26.33 | 31.87 | |
| 13.98 | 21.45 | 27.14 | 33.00 | |

13. Co-crystal according to claim 10 having an X-ray powder diffractogram as shown in Figure 1.

14. Co-crystal according to claim 10 having a monocline space group P21/c, unit cell dimensions a=10.9361(6) Å, b=9.5073(6) Å, c=31.209(2) Å, alpha= 90°, beta=93.8430(10)°, gamma=90° and a volume of 3237.6(3) A³.

15. Co-crystal according to claim 14 having an experimental diffractogram calculated from the X-ray diffractography on a single crystal as shown in Figure 2.

16. Pharmaceutical composition comprising the co-crystal according to any one of claims from 10 to 15 and a pharmaceutically acceptable carrier.

17. Co-crystal comprising fumaric acid and 3-benzyl-2-methyl-2,3,3a,4,5,6,7,7a-octahydrobenzo[d]isoxazol-4-one for use as a medicament.

18. Co-crystal comprising fumaric acid and 3-benzyl-2-methyl-2,3,3a,4,5,6,7,7a-octahydrobenzo[d]isoxazol-4-one for use in the treatment of mood disorders, disorders of anxiety, depression, convulsive conditions, in the improvement of learning ability, in the reversal of amnesia, in resolving abstinence syndrome from medicaments and drugs.
